# EUROPÄISCHE PATENTSCHRIFT

(11) **EP 2 316 416 B1**
(45) Veröffentlichungstag und Bekanntmachung des Hinweises auf die Patenterteilung: **23.12.2015**
(21) Anmeldenummer: 09174280.9
(22) Anmeldetag: 28.10.2009
(51) Int. Cl.: A61K 8/26, A61K 8/49, A61K 8/60, A61K 8/96, A61K 8/97, A61Q 17/04, A61Q 19/02

(54) **Hautaufhellende kosmetische Zusammensetzung**
Skin-lightening cosmetic compound
Composition cosmétique éclaircissant la peau

(43) Veröffentlichungstag der Anmeldung: 04.05.2011
(73) Patentinhaber: PM-International AG, 1618 Luxembourg (LU)
(72) Erfinder: Golz-Berner, Karin, 98000 Monaco (MC); Sorg, Rolf, L-5444 Schengen (LU); Meinhardt, Horst, D-56249 Herschbach (DE); Seifarth, Karin, D-60487 Frankfurt (DE)
(74) Vertreter: Gulde & Partner

(56) Entgegenhaltungen:
- EP-A1- 1 640 042
- WO-A2-2008/152159
- JP-A- 2009 137 846
- NIU H: "Preparation of beautifying health cosmetics containing special jewel, jade and medicinal stone fine powder" DERWENT, 5. Dezember 2001 (2001-12-05), XP002360136
- MANGUES M ET AL: "A new cosmetic active for safe skin brightening" SOFW-JOURNAL SEIFEN, OELE, FETTE, WACHSE, VERLAG FUR CHEMISCHE INDUSTRIE, AUGSBURG, DE, Bd. 134, Nr. 7, 1. Juli 2008 (2008-07-01), Seiten 52,54-58, XP009132007 ISSN: 0942-7694
- YUMING WEN ( YU, MING-WEN ) (YU, MING-WEN): 'The inhibition of tyrosinase activity of flavonoids in citrus peel' INTERNET CITATION, [Online] November 2008, Institute of Food Science, National Ilan University Gefunden im Internet: <URL:http://niufood.niu.edu.tw> [gefunden am 2012-06-26]
- DATABASE WPI Week 200238, 2002 Derwent Publications Ltd., London, GB; Class D21, AN 2002-349561 KIM J Y; YOON G S: 'COSMETIC COMPOSITION CONTAIN JADE POWDER COMPRISE MAGNESIUM OXIDE SILICON CALCIUM' & KR 2001 0 105 111 A ((SAIM-N) SAIMDANG CO LTD) 28 November 2001

## Beschreibung

Die Erfindung betrifft eine hautaufhellende kosmetische Zusammensetzung, ein kosmetisches Verfahren und ein Hautaufhellungs-Set.

Als kosmetische Aufheller sind seit längerer Zeit Kojisäure und Arbutin bekannt, für die umfangreiche Untersuchungsergebnisse veröffentlicht wurden. Die dabei gefundenen Unzulänglichkeiten wie Instabilität, Nebenwirkungen durch Hautirritationen, unzureichende Wirksamkeit haben dazu geführt, dass mit Hilfe verschiedener Pflanzenextrakte die Wirksamkeit verbessert werden sollte. In der EP 2092837 wird festgestellt, dass auch dabei Probleme auftraten hinsichtlich Stabilität und Farbänderungen. Der Zusatz von Vitamin C zeigte zwar auch Verbesserungswirkungen für die Hautaufhellung, hatte aber keine ausreichende Stabilität.

Reine Pflanzenextrakte wie der in WO 2009/069839 beschriebene Magnolia sieboldii-Extrakt zeigten erst nach 8 Wochen entsprechend wünschenswerte Aufhellungswirkungen.

Die WO 2009/105294 beschreibt eine Vielzahl von bekannten Hautaufhellern, die alle in "Assoziationsstrukturen" vorliegen sollen, d.h. die in liposomale, lamellare oder flüssigkristalline Strukturen eingebettet werden. Über dabei erzielte Ergebnisse wird bis auf einen Sting-Test nicht berichtet.

Die EP 1 640 041 A1 betrifft ein kosmetisches Aufhellungs- und Reinigungsmittel für die Haut, enthaltend Extrakte aus Lotus, Kiwi, Süßholz und Orchideen. In der WO 2008/152159 A2 wird die kosmetische Verwendung von 6-Hydroxy-7-methoxy-2,2-dimethylchroman und verwandten Stoffen zusammen mit einer Vielzahl von anderen hautaufhellenden Substanzen oder Pflanzenextrakten beschrieben. Die JP 2009-137846 beschreibt Hautaufhellungswirkungen von Blattextrakten der Rutaceae Citrus-Gruppe. CN 2002-189009 betrifft ein kosmetisches Mittel, das auch Hautaufhellung bewirken soll, bestehend aus Rubin-, Jade- und medizinischem Steinpulver von 1 nm bis 10 mm Korngröße, wobei der Jadeanteil bei 30 % liegt. Im SÖFW-Journal 134, 7-2008, S. 53-57 wird die hautaufhellende Wirkung von Chromabright® beschrieben.

Eine weitere Aufgabe besteht in der Bereitstellung eines kosmetischen Verfahrens zur Hautaufhellung unter Einsatz verschiedener Anwendungsformen der kosmetischen Zusammensetzung.

Eine weitere Aufgabe besteht darin, ein Set zur Verfügung zu stellen, mit dessen einzelnen Anwendungsformen der Zusammensetzung ein schneller und dauerhafter Aufhellungseffekt von dunkler Haut oder dunklen Flecken auf heller Haut erreicht werden kann.

Erfindungsgemäß umfasst eine hautaufhellende kosmetische Zusammensetzung kosmetische Hilfsstoffe und eine kombinatorische Einheit, bestehend aus gemahlenem weißen Jadestein mit einer mittleren Teilchengröße D₅₀ von 5 - 1 0 µm, Arbutin, einem Lotusblütenextrakt, einem Kumquat-Fruchtextrakt und Dimethylmethoxy Chroman Palmitate.

Dabei liegt die Konzentration des gemahlenen weißen Jadesteines im Bereich von 0,01 bis 0,5 Gew.-%, des Arbutins im Bereich von 0,1 bis 1,0 Gew.-%, des Lotusblütenextraktes im Bereich von 0,5 bis 1,5 Gew.-%, des Kumquat-Fruchtextraktes im Bereich von 0,1 bis 2,5 Gew.-% und des Dimethylmethoxy Chroman Palmitates im Bereich von 0,01 bis 0,1 Gew.-%, jeweils bezogen auf das Gesamtgewicht der kosmetischen Zusammensetzung.

Es wurde überraschend gefunden, dass die erfindungsgemäß kombinatorische Einheit nicht nur den zu erwartenden Aufhellungseffekt für die Haut zeigt, sondern dass
(a) ein wesentlicher Betrag der Hautaufhellung bereits nach sehr kurzer Anwendungszeit auftritt, z.B. nach 7 - 10 Tagen,
(b) der Aufhellungseffekt sehr lange anhält, z.B. nach 3-wöchiger Behandlung für einen weiteren Zeitraum von wenigstens 3 Wochen
(c) weder im Behandlungszeitraum noch danach merkliche Hautirritationen auftreten
(d) keine unerwünschten Farbtonänderungen im Behandlungszeitraum und danach auftreten.

Unter "kombinatorischer Einheit" wird das Gemisch der oben genannten 5 Bestandteile verstanden, die unverkapselt vorliegen, d.h. nicht in Liposomen oder lamellaren oder ähnlichen Strukturen eingeschlossen, und die bevorzugt als separat hergestelltes Gemisch in die kosmetische Zusammensetzung eingebracht wird.

Der Zusammenwirkungsmechanismus der 5 Bestandteile konnte bisher noch nicht aufgeklärt werden. Eine Besonderheit besteht darin, dass es sich um eine Kombination wasserlöslicher Ingredienzen (Arbutin und Pflanzenextrakte), öllöslicher Ingredienzen (Chroman Palmitate) und unlöslicher Partikel (Jade) handelt. Ohne an eine Theorie gebunden zu sein, könnten auch die Vielzahl der Verbindungen in dem Kumquat-Fruchtextrakt eine besondere Rolle spielen, insbesondere was die Stabilität des Gesamtproduktes und die sehr schnelle Wirksamkeit betrifft. Eine weitere Besonderheit besteht darin, dass alle 5 Bestandteile in freier Form, also unverkapselt vorliegen.

Die Zusammensetzung der Erfindung enthält unter anderem einen gemahlenen weißen Jadestein. "Weiß" im Sinne der Erfindung ist ein Bereich von reinweiß bis zu einem hellen Grün und erfasst die Farben der RAL-Tabelle weißgrün (RAL 6019), lichtgrün (RAL 6027), pastelltürkis (RAL 6034), seidenmatt weiß (RAL 8020), cremeweiß (RAL 9001), grauweiß (RAL 9002) und signalweiß (RAL 9013). "RAL" ist eine eingetragene Marke der RAL gemeinnützigen GmbH des RAL Deutsches Institut für Gütesicherung und Kennzeichnung e.V. in Sankt Augustin, Deutschland.

"Teilchengröße D₅₀" bedeutet eine Gauss'sche Verteilungskurve der Teilchen, bei der wenigstens 50 % der Teilchen im Bereich 5 - 10 µm liegen und der Rest über und unter diesem Bereich. Das Pulver enthält jedoch keine Teilchen >40 µm, wobei der Anteil an Teilchen im Bereich von 30 bis 40 µm weniger als 3 % beträgt.

Bevorzugte Anteile des Jadepulvers liegen im Bereich von 0,05 - 0,5 Gew.-%, insbesondere 0,08 - 0,2 Gew.-%.

Die unter der Handels- und INCI-Bezeichnung Arbutin bekannte Hydrochinon-D-glucose (α-Arbutin) hat zwar bekannte hautaufhellende Effekte, wirkt allerdings nach Herstellerangaben auch bei Konzentrationen von 2 Gew.-% erst nach ca. 3 Monaten merklich bei z.B. Leberflecken. Die Wirkung bei verringerten Konzentrationen von vorzugsweise 0,1 - 0,8 Gew.-%, insbesondere 0,2 - 0,6 Gew.-%, ist allerdings zusammen mit den anderen Bestandteilen überraschend schnell, und die Stabilität des Arbutins ist deutlich länger als zu erwarten war.

Der Lotusblütenextrakt in der kombinatorischen Einheit ist ein Extrakt, erhalten bei 12 bis 30°C mit einem mehrwertigen Alkohol wie Propylenglycol. Bevorzugte Anteile des Extraktes liegen im Bereich von 0,6 - 1,3 Gew.-%, insbesondere 0,8 - 1,2 Gew.-%. Ein Handelsprodukt ist z.B. Hydroglycolic Extract of Lotus Flowers Decolorized von Greentech, Saint Beauzire, Frankreich.

Erfindungsgemäß wird auch ein Kumquat-Extrakt eingesetzt. Kumquat mit der lateinischen Bezeichnung Fortunella crassifolia, F. hindsii, F. japonica (Syn. Citrus japonica), F. margarita, F. obovata, zählt zur Familie der Rautengewächse und bildet pflaumenförmige Früchte. Der Extrakt der Früchte mit Schale wird mit einem mehrwertigen Alkohol wie z.B. Propylenglycol bei 12 bis 35°C gewonnen. Ein handelsüblicher Extrakt ist z.B. Citrus Japonica Fruit Extract (INCI) mit der Bezeichnung Kumquat Hydroglycolic Extract von Greentech, Saint Beauzire, Frankreich.

Dimethylmethoxy Chroman Palmitate (vorgeschlagene INCI-Bezeichnung) ist ein weißer pulverförmiger Feststoff mit Tyrosinaseaktivität-hemmender Wirksamkeit von Lipotec, Gavà-Barcelona, Spanien (Chromabright).

Die erfindungsgemäße Zusammensetzung umfasst weitere kosmetische Hilfsstoffe wie Sonnenschutzfilter, Antioxidationsmittel, Hyaluronsäure, Gelbildner, Emulgatoren, kosmetische Ether, kosmetische Ester, Öle, Tonmineralien und Gemische davon sowie Wasser, Konservierungsmittel, Duftstoffe, Farbpigmente, Feuchthaltemittel, weitere Pflanzenextrakte wie Kamillenextrakt, Verdickungsmittel, α-Hydroxysäuren, Peptide, Flavonoide, Gelbildner, mehrwertige und einwertige Alkohole und Silicate.

Zu den Sonnenschutzfiltern gehören UVA-Filter, UVB-Filter und Breitbandfilter, die allgemein organischer oder anorganischer Herkunft sein können. Besonders bevorzugt für die vorliegende Erfindung sind organische UVA- und UVB-Filter, wie beispielsweise Diethylamino Hydroxybenzoyl Hexyl Benzoate, Bezophenone-3, Benzophenone-4, Ethylhexyl Methoxycinnamate, Ethylhexyl Triazone, Octocrylene, Benzophenone-9, PABA, Homosalate, Phenylbenzimidazole Sulfonic Acid, Terephthalidene Dicamphor Sulfonic Acid, Butyl Methoxydibenzoylmethane, Benzophenone-5 (sodium salt), Ethylhexyl Dimethyl PABA, Methylene Bis-Benzotriazolyl Tetramethylbutylphenol, Bis-Ethylhexyloxyphenol Methoxyphenyl Triazine und Gemische davon.

Die Einsatzmengen werden vom Fachmann gewählt, um Sonnenschutzfaktoren (LSF oder SPF) im Bereich von 8 bis 30, vorzugsweise 10 bis 20 zu erreichen und einen ausgeglichenen Schutz gegen UVA- und UVB-Strahlen zu gewährleisten.

Als Antioxidationsmittel werden Vitamine wie Vitamin A, Vitamin C und/oder Vitamin E sowie deren Derivate wie Palmitate, Phosphate, Acetate verwendet. Weitere Antioxidationsmittel sind Carotin und Carotinoide, Aminosäuren, α-Hydroxysäuren, Harnsäure usw.

Bevorzugte Gelbildner sind z.B. Carbomer, Guar Gum, Xanthan Gum, Alginate, Polyvinylalkohol, Polyvinylpyrrolidon. Besonders bevorzugt ist Carbomer.

Als Emulgatoren werden oberflächenaktive Mittel verwendet, die eine stabile O/W- oder W/O-Emulsion gewährleisten, insbesondere eine Öl-in-Wasser-Emulsion (O/W). Der Anteil des oberflächenaktiven Mittels liegt dabei üblicherweise im Bereich von 0,01 bis 20 Gew.-%, vorzugsweise 0,1 - 5 Gew.-%. Zu bekannten nicht-ionischen Emulatoren gehören die der Reihe Steareth-2 bis Steareth-1 00, gebildet aus Stearylalkohol und unterschiedlichen Ethylenoxideinheiten von 2-100; Beheneth-5 bis Beheneth-30, gebildet aus Behenylalkohol und 5-30 Ethylenoxideinheiten; Ceteareth 2-100, gebildet aus Stearyl- und Cetylalkohol mit 2-100 Ethylenoxideinheiten; Ceteth 1-45, gebildet aus Cetylalkohol und 1-45 Ethylenoxideinheiten usw. Weitere Emulgatoren sind solche wie PEG glyceryl 4 oleate, PEG glyceryl stearate mit 3-1000 Ethylenglycoleinheiten.

Auch lipophile Emulgatoren können verwendet werden, wie z.B. Partialester von Polyolen wie Glycerinmonoester, von Zuckerestern wie Saccharosedistearat, Sorbitansesquioleat oder von Pentaerythrit.

Anionische Tenside sind beispielsweise lipophile Reste mit anionischen Gruppen wie Carboxylat-, Sulfat-, Sulfonat-, oder Phosphatgruppen. Dazu gehören Alkylsulfate in Form von Alkali-, Ammonium- oder Alkanolammoniumsalzen wie Sodium Laureth Sulfate.

Kationische Tenside sind insbesondere quarternäre Ammoniumverbindungen oder auch Esterquats.

Bevorzugte kosmetische Ester und Ether sind zum Beispiel Steareth-2, Steareth-21, Cetearyl Octanoate, Isopropyl Myristate, Neopentyl Glycol Dicaprylate, Stearyl Ether.

Als kosmetische Öle können bekannte Öle wie Mineralöle, Pflanzenöle, Silikonöle verwendet werden. Besonders bevorzugt sind Siliconöle wie Dimethicone, Cyclomethicone.

Zu den Tonmineralien gehören Smektite, Schichtsilikate, Kaolin, Talk, Bentonit, Montmorillonit. Sie werden insbesondere in einer kosmetischen Maske eingesetzt, gegebenenfalls zusammen mit einem Zeolith.

Mehrwertige Alkohole sind z.B. Glycerin, Propylenglycole; einwertige Alkohole sind z.B. Ethanol, Propanol, i-Propanol.

Die hautaufhellende kosmetische Zusammensetzung kann in der Anwendungsform als Creme, Gel, Lotion, Serum, Maske, Make-up oder Grundierung vorliegen, insbesondere als Tagescreme, Nachtcreme, Reinigungsgel, Serum und Maske. Bevorzugte Masken sind Gesichts- und Dekolletémasken.

Ein weiterer Gegenstand der Erfindung ist ein kosmetisches, nichttherapeutisches Verfahren zur Hautaufhellung, bei dem auf die Hautoberfläche täglich wenigstens eine erste Folge von kosmetischen Zusammensetzungen eines Sets aufgetragen wird und im Abstand von 10 - 16 Stunden eine zweite Folge von kosmetischen Zusammensetzungen aufgetragen wird, wobei die erste und die zweite Folge als Anwendungsformen ein Reinigungsgel, ein Serum und eine Creme umfassen, die jeweils neben kosmetischen Hilfsstoffen die kombinatorische Einheit enthalten, bestehend aus gemahlenem weißen Jadestein mit einer mittleren Teilchengröße D₅₀ von 5 - 10 µm, Arbutin, einem Lotusblütenextrakt, einem Kumquat-Fruchtextrakt und Dimethylmethoxy Chroman Palmitate.

Das Verfahren ist dahingehend bevorzugt, dass die Creme der ersten Folge eine Tagescreme ist, die zusätzlich UV-Filter enthält, und die Creme der zweiten Folge eine Nachtcreme ist, die keine UV-Filter enthält.

Vorzugsweise wird morgens nach Auftragen des Reinigungsgels z.B. auf die komplette Gesichtsfläche, Hals- und gegebenenfalls Schulter- und Dekolletebereich, einer Einwirkzeit von 1 bis 15 Minuten und nachfolgendem Waschen dieser Bereiche mit Wasser ein Serum aufgetragen und 1 - 15 Minuten, bevorzugt 8 - 15 Minuten, einwirken gelassen. Danach wird die Tagescreme aufgetragen.

Bevorzugt am Abend, also nach etwa 10 bis 14 Stunden, werden in gleicher Reihenfolge das Reinigungsgel, das Serum und die Nachtcreme aufgetragen.

Das Verfahren wird für wenigstens eine Woche durchgeführt, vorzugsweise für 1 bis 6 Wochen, insbesondere für 1 bis 4 Wochen.

In einer weiteren Ausführungsform des Verfahrens umfassen die Anwendungsformen zusätzlich eine Maske, die 1- bis 3-mal, vorzugsweise 2- bis 3-mal wöchentlich aufgetragen wird und die eine kombinatorische Einheit umfasst, bestehend aus gemahlenem weißen Jadestein mit einer mittleren Teilchengröße D₅₀ von 5 - 10 µm, Arbutin, einem Lotusblütenextrakt, einem Kumquat-Fruchtextrakt und Dimethylmethoxy Chroman Palmitate. Weiterhin enthalten sind für eine kosmetische Maske übliche Hilfsstoffe wie kosmetische Ester, Wasser, Silicate, Zeolith usw.

Ein weiterer Gegenstand der Erfindung ist ein kosmetisches Set zur Aufhellung der Haut, umfassend als Anwendungsformen eine Tagescreme, eine Nachtcreme, ein Reinigungsgel, ein Serum und gegebenenfalls eine Maske, wobei jede Anwendungsform kosmetische Hilfsstoffe und die kombinatorische Einheit enthält und die kombinatorische Einheit 0,01 bis 0,5 Gew.-% eines gemahlenen weißen Jadesteins mit einer Teilchengröße D₅₀ von 5 - 10 µm, 0,1 bis 1 Gew.-%, Arbutin, 0,5 bis 1,5 Gew.-% eines Extraktes von weißen Lotusblüten, 0,1 bis 2,5 Gew.-% eines Extraktes von Kumquatfrüchten und 0,01 bis 0,1 Gew.-% Dimethylmethoxy Chroman Palmitat enthält, jeweils bezogen auf das Gesamtgewicht der Anwendungsform.

Bevorzugt ist ein Set, das zusätzlich eine Maske enthält, die wie die anderen Bestandteile des Sets die kombinatorische Einheit umfasst.

Die Erfindung wird durch Beispiele erläutert. Alle Angaben sind in Gewichtsprozent, bezogen auf das Gesamtgewicht der jeweiligen Anwendungsform.

### Beispiel 1 Reinigungsgel

| **Phase A** | |
|---|---|
| Wasser | bis 100 % |
| Glycerine | 6,0 |
| Sodium Laureth Sulfate | 10,0 |
| Carbomer | 0,5 |

| **Phase B** | |
|---|---|
| Triethanolamine | 0,5 |

| **Phase C** | |
|---|---|
| Nelumbo Nucifera Flower Extract | 0,5 |
| Arbutin | 0,1 |
| Dimethylmethoxy Chroman Palmitate | 0,01 |
| Citrus Japonica Fruit Extract | 0,5 |
| Jade (Powder) | 0,01 |
| Konservierungsmittel | 0,8 |

Die Bestandteile der Phasen A und C werden separat bei 15-25°C miteinander unter Rühren vermischt. Anschließend werden nacheinander die Phasen B und C unter Rühren zur Phase A gegeben und homogenisiert.

### Beispiel 2 Serum

| | |
|---|---|
| Wasser | bis 100 % |
| Glycerine | 3,0 |
| Carbomer | 0,8 |
| Triethanolamin | 0,8 |
| Ethanol | 10.0 |
| Konservierungsmittel | 0,5 |
| Parfüm | 0,5 |
| Nelumbo Nucifera Flower Extract | 1,5 |
| Arbutin | 0,8 |
| Dimethylmethoxy Chroman Palmitate | 0,08 |
| Citrus Japonica Fruit Extract | 2,3 |
| Jade (Powder) | 0,08 |

Die Bestandteile werden in der angegebenen Reihenfolge unter Rühren bei Umgebungstemperatur (15-25°C) miteinander vermischt. Am Schluss wird die kombinatorische Einheit als Premix hinzugegeben.

### Beispiel 3 Tagescreme mit SPF 15

| **Phase A** | |
|---|---|
| Wasser | bis 100 % |
| Glycerine | 2,0 |
| Carbomer | 0,8 |

| **Phase B** | |
|---|---|
| Dimethicone | 2,0 |
| Shea Butter | 3,0 |
| Bis-Ethylhexyloxyphenol Methoxyphenyl Triazine (Tinosorb^{®} S) | 1,5 |
| Ethylhexyl Methoxycinnamate | 7,5 |
| Steareth-21 | 2,0 |

| **Phase C** | |
|---|---|
| Triethanolamine | 0,8 |

| **Phase D** | |
|---|---|
| Nelumbo Nucifera Flower Extract | 1,5 |
| Arbutin | 0,8 |
| Dimethylmethoxy Chroman Palmitate | 0,01 |
| Citrus Japonica Fruit Extract | 2,0 |
| Jade (Powder) | 0,1 |
| Hyaluronic Acid | 0,2 |
| Chamomilla Recutia Flower Extract | 0,5 |
| Konservierungsmittel | 0,8 |
| Parfüm | 0,5 |

Die Phasen A und B werden auf 70-75°C unter Rühren erhitzt, zusammengegeben und für ca. 10 Minuten homogenisiert. Die Phase C wird hinzugegeben und das Gemisch für ca. 5 Minuten homogenisiert. Nach dem Abkühlen auf ca. 40°C wird die separat bei Umgebungstemperatur hergestellte Phase D unter Rühren zugegeben und dann auf 30°C abgekühlt.

### Beispiel 3a Tagescreme II

Es wird wie im Beispiel 3 verfahren. Der Gehalt an Arbutin beträgt 0,5 %, an Dimethylmethoxy Chroman Palmitate 0,02 % und Lotusblütenextrakt 1,2 %.

### Beispiel 4 Nachtcreme

| **Phase A** | |
|---|---|
| Wasser | bis 100 % |
| Glycerine | 3,0 |
| Carbomer | 1,0 |

| **Phase B** | |
|---|---|
| Steareth-21 | 2,0 |
| Steareth-2 | 1,7 |
| Dimethicone | 5,0 |

| **Phase C** | |
|---|---|
| Triethanolamine | 1,0 |

| **Phase D** | |
|---|---|
| Nelumbo Nucifera Flower Extract | 0,5 |
| Arbutin | 0,9 |
| Dimethylmethoxy Chroman Palmitate | 0,01 |
| Citrus Japonica Fruit Extract | 2,0 |
| Jade (Powder) | 0,5 |
| Konservierungsmittel | 0,8 |
| Chamomilla Recutia Flower Extract | 1,0 |
| Parfüm | 0,5 |

Die Herstellung erfolgt wie in Beispiel 3.

### Beispiel 5 Maske

| **Phase A** | |
|---|---|
| Wasser | bis 100 % |
| Zeolite | 1,0 |
| Glycerine | 3,0 |
| Bentonite | 3,0 |
| Kaolin | 35,0 |
| Talc | 3,0 |

| **Phase B** | |
|---|---|
| Propylene Glycol Hydroxystearate | 2,0 |
| Isopropyl Lanolate | 3,0 |
| Konservierungsmittel | 1,0 |

| **Phase C** | |
|---|---|
| Nelumbo Nucifera Flower Extract | 1,0 |
| Arbutin | 0,5 |
| Dimethylmethoxy Chroman Palmitate | 0,05 |
| Citrus Japonica Fruit Extract | 0,5 |
| Jade (Powder) | 0,1 |
| Chamomilla Recutia Flower Extract | 0,5 |
| Camellia Sinensis Leaf Extract | 0,1 |
| Parfüm | 1,0 |

Die Bestandteile der Phase A werden bei 70°C unter Rühren vermischt und ca. 20 homogenisiert, wobei die gleichmäßige Pigmentverteilung geprüft wird. Die Bestandteile der Phase B werden unter Rühren auf 70°C erhitzt, und die Phasen A und B werden zusammengeführt, wobei wenigstens 15 Minuten homogenisiert wird. Nach dem Abkühlen auf 40-45°C wird die Phase mit dem Premix der kombinatorischen Einheit unter Rühren zugegeben und es wird nochmals homogenisiert.

### Beispiel 6 Verfahren

Von 12 Probanden (weiblich, Alter 22-43 Jahre) mit dem Hauttyp "Tan" oder "Matt" (Chardon et al., Int. J. Cosm. Scien. 13, 191-208, 1991) (entspricht in einer anderen Klassifizierung dem Hauttyp "Mediterran dunkel, asiatisch") wurde das Reinigungsgel gemäß Beispiel 1 am Morgen auf die Gesichtshaut durch intensiven, reibenden Druck über 2 Minuten aufgetragen. Danach wurde die Gesichtshaut mit Wasser abgespült.

Das Serum gemäß Beispiel 2 wurde gleichmäßig in die Gesichtshaut einmassiert und 10 Minuten einwirken gelassen. Danach wurde die Tagescreme gemäß Beispiel 3 gleichmäßig auf der Gesichtshaut verteilt.

Die Probanden waren teilweise am Tage auch direkter Sonnenstrahlung ausgesetzt, jedoch nicht länger als insgesamt 2 Stunden. Nach 12 Stunden wurde in gleicher Weise wie am Morgen das Reinigungsgel und das Serum aufgebracht und danach die Nachtcreme gemäß Beispiel 4.

Der Verfahrensablauf wurde täglich über einen Zeitraum von 21 Tagen wiederholt. An den Tagen 5, 10 und 14 wurde zwischen der Anwendung am Morgen und am Abend im Gesicht eine Maske gemäß Beispiel 5 aufgetragen. Die Einwirkungszeit der Maske betrug 10 Minuten. Danach wurde die Maske mit Wasser abgespült.

Die Auswertung erfolgte an den Tagen 0, 7, 14 und 21 durch erfahrene Tester mittels Farbkarten. Die Farbkarten zeigten 10 Brauntöne in Abstufungen von jeweils 10 % Veränderung mit jedem Braunton. Die Ergebnisse sind in Tabelle 1 dargestellt.

**Tabelle 1**

| Messung nach [Tage] | Aufhellung [%] | Anteil der Probanden [%] |
|---|---|---|
| 3 | 20 | 65 |
| 7 | 40-50 | 75 |
| | 30 | 25 |
| 14 | 60-70 | 83 |
| | 50 | 17 |
| 21 | 90 | 25 |
| | 70-80 | 58 |
| | 60 | 17 |
| 42 | 90 | 17 |
| | 80 | 33 |
| | 60 | 42 |
| | 50 | 8 |

Die erneute Messung nach 42 Tagen nach Testbeginn erfolgte, ohne dass nach dem 21. Tag eine Behandlung vorgenommen wurde.

Die Ergebnisse zeigen eine sehr schnelle Wirksamkeit der Behandlung, da bereits nach 3 Tagen sichtbare Aufhellungsergebnisse erkennbar waren und bei der Mehrzahl der Probanden nach 7 Tagen schon bis zu 50 % Aufhellung gefunden wurden.

Die Nachhaltigkeit der Aufhellung zeigt das Ergebnis nach 21 Tagen, wonach der Rückgang der Aufhellung ohne Behandlung nur sehr zögernd einsetzt und die Mehrzahl der Probanden noch im Bereich 60 - 80 % liegt.

Es wurden keine unerwünschten Farbtonänderungen oder Hautirritationen festgestellt.

### Beispiel 7 Vergleich

Ein Vergleichsversuch mit ebenfalls 12 Probanden (weiblich, Alter 25 - 44 Jahre) mit dem Hauttyp "Tan" bis "Matt" wurde in ähnlicher Weise wie im Beispiel 6 durchgeführt. Ausnahme: Die jeweiligen kosmetischen Anwendungsformen Reinigungsgel, Serum, Cremes und Maske enthielten die kombinatorische Einheit ohne den Bestandteil Kumquat-Fruchtextrakt, der in den Gesamtzusammensetzungen durch einen höheren Anteil Wasser ersetzt wurde.

In einer verkürzten Auswertung nach 3, 7, 21 und 42 Tagen wurden die Ergebnisse gemäß Tabelle 2 erzielt.

**Tabelle 2**

| Messung nach [Tage] | Aufhellung [%] | Anteil der Probanden [%] |
|---|---|---|
| 3 | 0 | 100 |
| 7 | 10 | 25 |
| 21 | 40 | 25 |
| | 30 | 58 |
| | 10 | 17 |
| 42 | 30 | 25 |
| | 10 | 66 |
| | 0 | 9 |

Die Ergebnisse zeigen, dass die schnellere Wirksamkeit der erfindungsgemäßen Zusammensetzungen nicht erreicht wird und auch die länger anhaltende Wirkung nur bei einem kleinen Teil der Probanden und in geringerer Höhe.

## Patentansprüche

1. Hautaufhellende kosmetische Zusammensetzung, umfassend kosmetische Hilfsstoffe und eine kombinatorische Einheit, bestehend aus 0,01 bis 0,5 Gew.-% gemahlenem weißen Jadestein mit einer mittleren Teilchengröße D₅₀ von 5 - 10 µm;
Arbutin im Bereich von 0,1 bis 1,0 Gew.-%; einem Lotusblütenextrakt im Bereich von 0,5 bis 1,5 Gew.-%, erhalten bei 12-30°C mit einem mehrwertigen Alkohol; einem Kumquat-Fruchtextrakt im Bereich von 0,1 bis 2,5 Gew.-%, erhalten bei 12-35°C mit einem mehrwertigen Alkohol aus den Früchten mit Schale; und von Dimethylmethoxy Chroman Palmitate im Bereich von 0,01 bis 0,1 Gew.-%, jeweils bezogen auf das Gesamtgewicht der kosmetischen Zusammensetzung.

2. Zusammensetzung nach Anspruch 1, **dadurch gekennzeichnet, dass** sie als kosmetische Hilfsstoffe Sonnenschutzfilter, Antioxidationsmittel, Hyaluronsäure, Gelbildner, Emulgatoren, kosmetische Ether, kosmetische Ester, Öle, Tonmineralien und Gemische davon umfasst.

3. Zusammensetzung nach Anspruch 1 oder 2, **dadurch gekennzeichnet, dass** sie als Maske, Creme, Serum oder Gel vorliegt.

4. Kosmetisches, nicht-therapeutisches Verfahren zur Hautaufhellung, **dadurch gekennzeichnet, dass** auf die Hautoberfläche täglich wenigstens eine erste Folge von kosmetischen Zusammensetzungen eines Sets aufgetragen wird und im Abstand von 10 - 16 Stunden eine zweite Folge von kosmetischen Zusammensetzungen aufgetragen wird, wobei die erste und die zweite Folge als Anwendungsformen ein Reinigungsgel, ein Serum und eine Creme umfassen, die jeweils neben kosmetischen Hilfsstoffen eine kombinatorische Einheit gemäß Anspruch 1 oder 2 enthalten.

5. Verfahren nach Anspruch 4, **dadurch gekennzeichnet, dass** die Creme der ersten Folge eine Tagescreme ist, die zusätzlich UV-Filter enthält, und die Creme der zweiten Folge eine Nachtcreme ist, die keine UV-Filter enthält.

6. Verfahren nach einem der Ansprüche 4 bis 5, **dadurch gekennzeichnet, dass** es für wenigstens eine Woche durchgeführt wird, vorzugsweise für 1-4 Wochen.

7. Verfahren nach Anspruch 4, **dadurch gekennzeichnet, dass** die Anwendungsformen zusätzlich eine Maske umfassen, die 1- bis 3-mal wöchentlich aufgetragen wird und die eine kombinatorische Einheit gemäß Anspruch 1 oder 2 umfasst.

8. Kosmetisches Set zur Aufhellung der Haut, umfassend als Anwendungsformen eine Tagescreme, eine Nachtcreme, ein Reinigungsgel, ein Serum und gegebenenfalls eine Maske, wobei jede Anwendungsform kosmetische Hilfsstoffe und eine kombinatorische Einheit gemäß Anspruch 1 enthält.

## Claims

1. A skin-lightening cosmetic composition comprising cosmetic auxiliaries and a combinatory unit consisting of 0.01 to 0.5 % by weight of ground white jade stone with an average particle size D₅₀ of 5-10 µm;
arbutin in the range from 0.1 to 1.0 % by weight; a lotus flower extract in the range from 0.5 to 1.5 % by weight, obtained at 12-30°C using a polyvalent alcohol;
a kumquat fruit extract in the range from 0.1 to 2.5 % by weight, obtained at 12-35°C from the fruits including the skin using a polyvalent alcohol; and Dimethylmethoxy Chroman Palmitate in the range from 0.01 to 0.1 % by weight, each relative to the total weight of the cosmetic composition.

2. The composition according to claim 1, **characterized in that**, as cosmetic auxiliaries, it comprises sun filters, antioxidants, hyaluronic acid, gel-forming agents, emulsifiers, cosmetic ethers, cosmetic esters, oils, clay minerals and mixtures thereof.

3. The composition according to Claim 1 or 2, **characterized in that** it is provided as a mask, cream, serum or gel.

4. A cosmetic, non-therapeutic method for skin-lightening, **characterized in that** daily at least one first series of cosmetic compositions of a set are applied to the skin surface and 10-16 hours later a second series of cosmetic compositions are applied, wherein the first and the second series comprise, as forms of application, a cleansing gel, a serum and a cream, each of which contains a combinatory unit according to Claim 1 or 2 and cosmetic auxiliaries.

5. The method according to Claim 4, **characterized in that** the cream of the first series is a day cream which additionally contains UV filters, and the cream of the second series is a night cream which does not contain any UV filters.

6. The method according to any one of Claims 4 to 5, **characterized in that** it is carried out for at least one week, preferably for 1-4 weeks.

7. The method according to Claim 4, **characterized in that** the forms of application additionally comprise a mask which is applied 1 to 3 times per week and which comprises a combinatory unit according to Claim 1 or 2.

8. A cosmetic set for lightening the skin, comprising, as forms of application, a day cream, a night cream, a cleansing gel, a serum and optionally a mask, wherein each form of application contains cosmetic auxiliaries and a combinatory unit according to Claim 1.

## Revendications

1. Composition cosmétique éclaircissant la peau, comprenant des agents auxiliaires cosmétiques et une unité combinatoire, se composant de 0,01 à 0,5 % massique de pierre de jade blanc moulue, avec une taille de particule moyenne D₅₀ de 5 à 10 µm ; de l'arbutine dans une proportion de 0,1 à 1,0 % massique ; un extrait de fleur de lotus dans une proportion de 0,5 à 1,5 % massique, obtenu à 12-30 °C avec un polyalcool ;
un extrait de fruit de kumquat dans une proportion de 0,1 à 2,5 % massique, obtenu à 12-35 °C avec un polyalcool, à partir des fruits avec écorce ; et de diméthylméthoxy chroman palmitate dans une proportion de 0,01 à 0,1 % massique, se rapportant respectivement au poids total de la composition cosmétique.

2. Composition selon la revendication 1, **caractérisée en ce qu'**elle comprend des filtres solaires, agents anti-oxydants, acide hyaluronique, agents gélifiants, émulsifiants, éthers cosmétiques, esters cosmétiques, huiles, minéraux argileux et mélanges de ceux-ci, comme agents auxiliaires cosmétiques.

3. Composition selon la revendication 1 ou 2, **caractérisée en ce qu'**elle existe comme masque, crème, sérum ou gel.

4. Procédé cosmétique, non thérapeutique, pour l'éclaircissement de la peau, **caractérisé en ce qu'**au moins une première séquence de compositions cosmétiques d'un set est appliquée sur la surface de la peau et une deuxième séquence de compositions cosmétiques est appliquée après un intervalle de 10 à 16 heures, la première et la deuxième séquences comprenant comme formes d'application un gel nettoyant, un sérum et une crème, comprenant respectivement, en plus des agents auxiliaires cosmétiques, une unité combinatoire selon la revendication 1 ou 2.

5. Procédé selon la revendication 4, **caractérisé en ce que** la crème de la première séquence est une crème de jour, comprenant de plus des filtres UV, et **en ce que** la crème de la deuxième séquence est une crème de nuit, ne comprenant aucun filtre UV.

6. Procédé selon l'une des revendications 4 à 5, **caractérisé en ce qu'**il est appliqué pendant au moins une semaine, de préférence de 1 à 4 semaines.

7. Procédé selon la revendication 4, **caractérisé en ce que** les formes d'application comprennent de plus un masque, lequel est appliqué de 1 à 3 fois par semaine et comprend une unité combinatoire selon la revendication 1 ou 2.

8. Set cosmétique pour l'éclaircissement de la peau, comprenant comme formes d'application une crème de jour, une crème de nuit, un gel nettoyant, un sérum et, le cas échéant, un masque, chaque forme d'application comprenant des agents auxiliaires cosmétiques et une unité combinatoire selon la revendication 1.
